(19) Europäisches Patentamt
European Patent Office
Office européen des brevets

(11) Veröffentlichungsnummer: **0 649 838 A1**

(12) **EUROPÄISCHE PATENTANMELDUNG**

(21) Anmeldenummer: **94115921.2**

(22) Anmeldetag: **10.10.94**

(51) Int. Cl.[6]: **C07D 205/04**, C07D 207/08, C07D 207/12, C07D 211/22, C07D 211/42, C07D 211/46, C07D 223/08, A61K 31/445, A61K 31/40, A61K 31/55

(30) Priorität: **20.10.93 DE 4335718**

(43) Veröffentlichungstag der Anmeldung:
**26.04.95 Patentblatt 95/17**

(84) Benannte Vertragsstaaten:
**AT BE CH DE DK ES FR GB GR IE IT LI LU NL PT SE**

(71) Anmelder: **MERCK PATENT GmbH**
**Frankfurter Strasse 250**
**D-64293 Darmstadt (DE)**

(72) Erfinder: **Baumgarth, Dr. Manfred**
**Sachsenstrasse 53**
**D-64297 Darmstadt (DE)**
Erfinder: **Lues, Dr. Inge**
**Katharinenstrasse 2**
**D-64297 Darmstadt (DE)**
Erfinder: **Minck, Dr. Klaus-Otto**
**Büchenstrasse 6**
**D-64372 Ober-Ramstadt (DE)**

(54) **N-Phenylalkyl/Phenoxyalkyl-Azetidin/Pyrrozidin/Piperidin/Hexahydroazepin-Derivate als antiarrhythmische Wirkstoffe.**

(57) Cyclische Aminderivate der Formel I

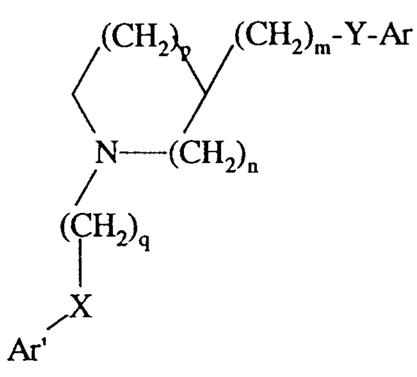

I,

worin

| | |
|---|---|
| Ar und Ar' | jeweils unabhängig voneinander unsubstituiertes oder ein- oder zweifach durch $NO_2$, $NH_2$, Hal, $CF_3$, A, $NHSO_2A$ oder NHAc substituiertes Phenyl, |
| X und Y | jeweils unabhängig voneinander O oder eine Bindung, |
| m | 0 oder 1, |
| n | 0, 1 oder 2, |
| p | 0, 1, 2 oder 3, |
| q | 2 oder 3, |
| A | Alkyl mit 1-6 C-Atomen, |

EP 0 649 838 A1

| | |
|---|---|
| Hal | F, Cl, Br oder I und |
| Ac | Alkanoyl mit 1-8 C-Atomen, Aralkanoyl mit 8-10 C-Atomen oder Aroyl mit 7-11 C-Atomen bedeuten, sowie deren physiologisch unbedenkliche Salze, zeigen antiarrhythmische Wirkungen. |

Die Erfindung betrifft neue cyclische Aminderivate der Formel I

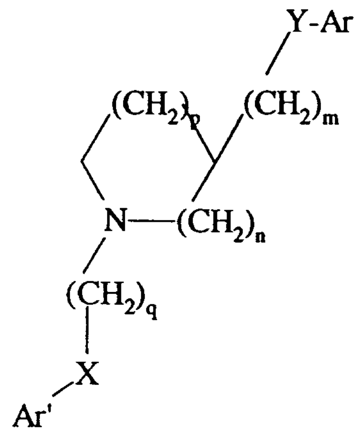

I,

worin

| | |
|---|---|
| Ar und Ar' | jeweils unabhängig voneinander unsubstituiertes oder ein- oder zweifach durch $NO_2$, $NH_2$, Hal, $CF_3$, A, $NHSO_2A$ oder NHAc substituiertes Phenyl, |
| X und Y | jeweils unabhängig voneinander O oder eine Bindung, |
| m | 0 oder 1, |
| n | 0, 1 oder 2, |
| p | 0, 1, 2 oder 3, |
| q | 2 oder 3, |
| A | Alkyl mit 1-6 C-Atomen, |
| Hal | F,Cl, Br oder I und |
| Ac | Alkanoyl mit 1-8 C-Atomen, Aralkanoyl mit 8-10 C-Atomen oder Aroyl mit 7-11 C-Atomen |

bedeuten,
sowie deren physiologisch unbedenkliche Salze.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze bei guter Verträglichkeit wertvolle pharmakologische Eigenschaften besitzen. So zeigen sie insbesondere antiarrhythmische bzw. die Refraktärzeit des Herzens verlängernde, positiv inotrope Wirkungen.

Die Herzwirkung kann z.B. an narkotisierten oder wachen Ratten, Meerschweinchen, Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1a (1981), Seiten 141 bis 170, oder von Schliep et al. im 9th International Congress of Pharmacol., London (1984), Abstracts of papers 9P, beschrieben sind.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe dienen.

Gegenstand der Erfindung sind dementsprechend die Verbindungen der Formel I, ihre Säureadditionssalze sowie ein Verfahren zu ihrer Herstellung, dadurch gekennzeichnet, daß man eine Verbindung der Formel II

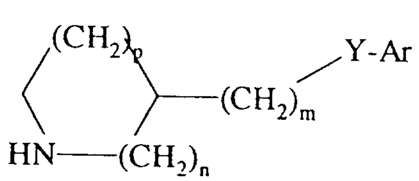

II,

worin
Ar, Y, m, n und p die angegebene Bedeutung haben,
mit einer Verbindung der Formel III,

Ar-X-$(CH_2)_q$-L III,

worin
Ar, X und q die angegebene Bedeutung haben, und
L OH, Cl, Br oder eine reaktionsfähige, funktionell abgewandelte OH-Gruppe bedeutet,
umsetzt,
oder daß man zur Herstellung einer Verbindung der Formel I gemäß Anspruch 1, in der Y Sauerstoff bedeutet, eine Verbindung der Formel IV

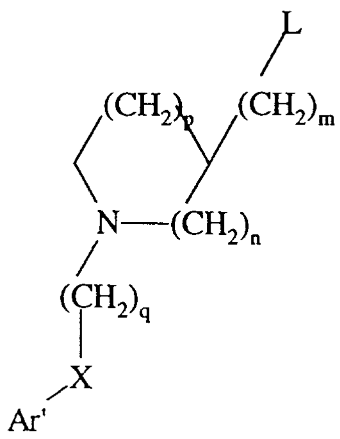

IV,

worin
Ar', X, L, m, n, p und q die angegebenen Bedeutungen besitzen,
mit einer Verbindung der Formel V

Ar-Z V,

worin
Ar die angegebene Bedeutung hat, und
Z OH oder eine reaktionsfähige, funktionell abgewandelte OH-Gruppe, einschließlich einer Gruppe mit salzartigem Charakter bedeutet,
umsetzt,
oder daß man eine Verbindung der Formel Va

Ar'-Z Va,

worin Ar' und Z die angegebenen Bedeutungen haben,
mit einer Verbindung der Formel VI

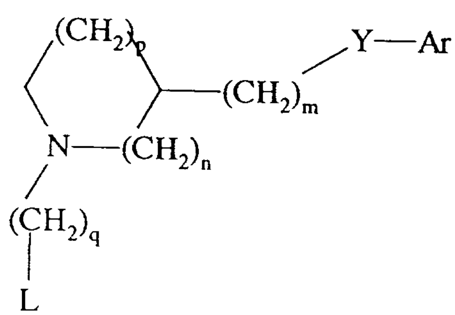

VI,

worin
Ar, Y, L, m, n, p und q die angegebenen Bedeutungen haben, umsetzt,
oder daß man eine Verbindung, die an sich der Formel I entspricht, aber anstelle einer oder mehrerer $CH_2$-

Gruppen eine oder mehrere reduzierbare Gruppen besitzt, durch Reduktion in eine Verbindung der Formel I überführt und/oder daß man in einer Verbindung der Formel I eine oder beide Gruppen Ar bzw. Ar' in andere Reste Ar bzw. Ar' umwandelt und/oder daß man eine basische Verbindung der Formel I durch Behandeln mit einer Säure in eines ihrer physiologisch unbedenklichen Säureadditionssalze umwandelt.

Vor- und nachstehend haben Ar, Ar', A, Hal, L, X, Y und Z sowie die Parameter m, n, p und q die bei den Formeln I, III und V angegebenen Bedeutungen, wenn nicht ausdrücklich etwas anderes angegeben ist.

Der Rest A bedeutet Alkyl mit 1, 2, 3, 4, 5 oder 6, insbesondere 1, 2 oder 3 C-Atomen, vorzugsweise Methyl, ferner auch Ethyl, n-Propyl, Isopropyl, n-Butyl, Isobutyl, sek.-Butyl oder tert.-Butyl. In $NHSO_2A$ ist A vorzugsweise Methyl.

Die Gruppe Ac steht vorzugsweise für Alkanoyl mit 1-8 C-Atomen, insbesondere mit 1, 2, 3, 4 oder 5 C-Atomen; im einzelnen bedeutet Ac bevorzugt Acetyl, ferner bevorzugt Formyl, Propionyl, Butyryl, Isobutyryl, Valeryl, Isovaleryl, Pivaloyl (Trimethylacetyl), weiterhin bevorzugt unsubstituiertes oder gegebenenfalls substituiertes Aroyl mit 7-11 C-Atomen, wobei als Substituenten insbesondere 1-3, vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, I, $NO_2$, $NH_2$, Alkylamino oder Dialkylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4- oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylthiobenzoyl, o-, m- oder p-Methylsulfinylbenzoyl, o-, m- oder p-Methylsulfonylbenzoyl, 2,3- oder 3,4-Methylendioxybenzoyl, 1- oder 2-Naphthoyl. Ac kann weiterhin für Aralkanoyl mit 1-10 C-Atomen wie z.B. Phenylacetyl, 2- oder 3-Phenylpropionyl oder 2-, 3- oder 4-Phenylbutyryl stehen.

NHAc bedeutet besonders bevorzugt Acetamido.

Falls eine der Verbindungen der Formeln I bis VI mehrere Gruppen A und/oder Ac enthält, so können die Gruppen des jeweils gleichen Typs gleich oder voneinander verschieden sein.

Die Reste Ar und Ar' stehen jeweils unabhängig voneinander für unsubstituiertes oder ein- bzw. zweifach substituiertes Phenyl, vorzugsweise weisen die beiden Reste jedoch das gleich Substitutionsmuster auf. Sofern sie einfach substituiert sind, steht der entsprechende Substituent bevorzugt in para-Position. Besonders bevorzugte Substituenten am Phenylrest sind $-NH_2$, $-NO_2$, $-NHSO_2CH_3$ oder $-NHCOCH_3$.

Y bedeutet vorzugsweise Sauerstoff, während X bevorzugt für eine Bindung steht.

Die Variable m ist vorzugsweise 1, q ist bevorzugt 2, wohingegen n besonders bevorzugt 1 oder 0 und p 1, 2 oder 3 ist.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat.

Einige bevorzugte Gruppen von Verbindungen können durch die folgenden Teilformeln Ia bis Ig ausgedrückt werden, die der Formel I entsprechen und worin die nicht näher bezeichneten Reste und Parameter die bei der Formel I angegebene Bedeutung haben, worin jedoch

- in Ia Ar und Ar' jeweils p-Methylsulfonamidophenyl bedeuten;
- in Ib Ar und Ar' jeweils p-Nitrophenyl bedeuten;
- in Ic Ar und Ar' jeweils p-Aminophenyl bedeuten;
- in Id $n = 2$ und $p = 1$ bedeuten, so daß die heterocyclische Gruppe einen Piperidin-Rest darstellt;
- in Ie $n = 1$ und $p = 3$ bedeuten, so daß die heterocyclische Gruppe einen Hexahydroazepin-Rest darstellt;
- in If $n = 0$ und $p = 2$ bedeuten, so das die heterocyclische Gruppe einen Pyrrolidin-Rest darstellt;
- in Ig $q = 2$ und $m = 0$ bedeuten und n sowie p die in Id bis If angegebenen Bedeutungen besitzen.

Ferner sind bevorzugt Verbindungen der Teilformeln Ih sowie Iah bis Igh, die den Teilformeln I sowie Ia bis Ig entsprechen, worin jedoch zusätzlich Y Sauerstoff und X eine Bindung bedeutet.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart oder J. March, Adv. Org. Chem., 3rd Ed., J. Wiley & Sons, N.Y. (1985)) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Ausgangsstoffe der Formel II und III sind teilweise bekannt. Sofern sie nicht bekannt sind, können sie nach an sich bekannten Methoden hergestellt werden. Die Herstellung von Verbindungen der Formel II

in denen Y Sauerstoff ist, erfolgt beispielsweise ausgehend von Verbindungen, die an sich der Formel II entsprechen, aber anstelle des Restes -Y-Ar z.B. eine OH-Gruppe, eine reaktionsfähige funktionell abgewandelte OH-Gruppe, Cl oder Br enthalten und deren sekundäres N-Atom durch eine an sich bekannte Schutzgruppe blockiert ist, durch Umsetzung mit Phenol oder einem substituierten Derivat bzw. einem entsprechenden Phenolat unter Bedingungen wie sie für die Ethersynthese an sich bekannt sind, insbesondere aber unter den Bedingungen der Mitsonubo-Reaktion (J. Am. Chem. Soc. 104, 6876 (1982)).

Ferner können bestimmte Verbindungen der Formel II (Y = eine Bindung) beispielsweise auch ausgehend von Benzylpyrrolidinen oder -piperidinen bzw. Phenylpyrrolidinen oder -piperidinen durch Substitution am Aromaten, insbesondere Nitrierung, gegebenenfalls mit nachfolgender Reduktion und weiterer Derivatisierung, hergestellt werden.

Die substituierten Phenylverbindungen der Formel II sind in der Regel bekannt oder leicht in Analogie zu den bekannten herstellbar.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Alkali- oder Erdalkalimetall-Salzes einer schwachen Säure, vorzugsweise des Kaliums, Natriums oder Calciums, oder der Zusatz einer organischen Base wie Triethylamin, Dimethylanilin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, daß man zur Herstellung einer Verbindung der Formel I, in der Y Sauerstoff bedeutet, eine Verbindung der Formel IV mit einem Benzolderivat der Formel V umsetzt.

Verbindungen der Formel IV sind z.B. zugänglich, indem man cyclische Amine wie beispielsweise Piperidinole, Pyrrolidinole, Prolinole oder aber cyclische Amine, die durch eine Hydroxymethyl- oder Halogenmethylgruppe, wobei Halogen vorzugsweise Chlor oder Brom bedeutet, substituiert sind, mit einem Phenalkylbromid oder -chlorid (Alkyl = Ethyl oder Propyl) bzw. Phenoxyalkylbromid oder -chlorid, wobei das aromatische System auch durch einen oder zwei der für Ar angegebenen Reste substituiert sein kann, umsetzt, so daß eine N-Alkylierung erfolgt.

Die Benzolderivate der Formel V sind in der Regel bekannt und können nach den Methoden, die für die Substitution von Aromaten an sich bekannt sind, hergestellt werden.

Die Umsetzung der Verbindungen der Formel IV mit denen der Formel V erfolgt unter den Bedingungen wie sie für die Synthese von Ethern typisch sind. Als Lösungsmittel eignen sich die zuvor für die Umsetzung von II mit III genannten. Ebenso kommen die gleichen Reaktionszeiten und -temperaturen in Frage. Besonders bevorzugte Reaktionsbedingungen sind die der Mitsonubo-Reaktion, unter Verwendung von Azodicarbonsäurediester und Triphenylphosphin, sofern eine Verbindung der Formel I mit Y = Sauerstoff hergestellt werden soll.

Bemerkenswert ist es, daß es bei der Umsetzung von IV mit V unter "Mitsonubo-Bedingungen" zu einer Umlagerung unter Ringverengung oder Ringerweiterung um jeweils ein C-Atom des cyclischen Aminbausteins kommen kann, so daß beispielsweise aus einem Pyrrolidin- ein Piperidinderivat oder aus einem Piperidin- ein Hexahydro-azepinderivat, aber auch in umgekehrter Weise aus einem Piperidin- ein Pyrrolidin-System entstehen kann.

Entstandene Produktgemische zwischen fünf- und sechsgliedrigen Ringen bzw. sechs- und siebengliedrigen Heterocyclen können leicht durch präparative Chromatographie an Kieselgel getrennt werden.

Weiterhin können Verbindungen der Formel I auch durch Umsetzung von Benzolderivaten der Formel Va mit cyclischen Aminen der Formel VI herstellt werden. Die Verbindungen der Formel Va sind an sich bekannt oder in Analogie zu an sich bekannten, beispielsweise nach den Methoden der elektrophilen Substitution am Aromaten, herstellbar.

Verbindungen der Formel VI können beispielsweise ausgehend von dem jeweiligen cyclischen Amin durch Veretherung der freien Hydroxygruppe der Seitenkette sowie N-Alkylierung des Heterocyclus, gegebenenfalls nach Abspaltung einer notwendigen Schutzgruppe unter den Bedingungen wie sie zur Herstellung der Verbindungen der Formel II mit denen der Formel III oben angegeben werden, hergestellt werden.

Die Umsetzung der Verbindungen Va mit den Substanzen der Formel VI erfolgt in Analogie zu der Umsetzung von IV mit V wie zuvor angegeben, wobei in diesem Fall keine Umlagerungen auftreten.

Ferner ist es möglich, eine Verbindung der Formel I zu erhalten, indem man eine Verbindung, die an sich der Formel I entspricht, aber anstelle einer oder mehrerer $CH_2$-Gruppen eine oder mehrere reduzierbare Gruppen enthält, reduziert, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen enthalten, reduktiv in eine Verbindung der Formel I zu überführen. Vorzugsweise bedient man sich hierzu des nascierenden Wasserstoffs oder komplexer Metallhydride, ferner der Reduktionen mit Wasserstoffgas unter Übergangsmetallkatalyse.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung ist zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich die -CO-Gruppen in den Säureamiden mit $LiAlH_4$ in THF bei Temperaturen zwischen etwa 0 und 66° zu $CH_2$-Gruppen reduzieren.

Darüber hinaus ist es möglich, bestimmte Reduktionen durch Verwendung von $H_2$-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen beispielsweise durch katalytische Hydrierung mit $Pd/H_2$ in Methanol in $NH_2$-Gruppen umgewandelt werden.

Weiterhin kann man eine Verbindung der Formel I nach an sich bekannten Methoden in eine andere Verbindung der Formel I umwandeln.

Die Phenylringe der Verbindungen der Formel I können beispielsweise, sofern Nebenreaktionen auszuschließen sind, unter den Bedingungen der Friedel-Crafts-Reaktionen chloriert, bromiert oder alkyliert werden, indem man das entsprechende Halogen oder Alkylchlorid bzw. Alkylbromid unter Katalyse von Lewis-Säuren, wie z.B. $AlCl_3$, $FeBr_3$ oder Fe, bei Temperaturen zwischen 30° und 150°, zweckmäßig zwischen 50° und 150° in einem inerten Lösungsmittel, wie z.B. Kohlenwasserstoffen, THF oder Tetrachlorkohlenstoff mit der zu derivatisierenden Verbindung der Formel I umsetzt.

Ferner ist es möglich, daß man eine Verbindung der Formel I, in der Ar oder Ar' durch $NH_2$ substituiert ist, durch Alkylierung oder Acylierung, nach Methoden wie sie für Amine allgemein üblich und bekannt sind, in entsprechende Verbindungen der Formel I, in denen Ar bzw. Ar' durch NHA oder NHAc substituiert ist, umwandelt.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen.

Die Verbindungen der Formel I können ein Asymmetriezentrum besitzen. Sie können daher bei ihrer Herstellung als Racemate oder, falls optisch aktive Ausgangsstoffe verwendet werden, auch in optisch aktiver Form erhalten werden. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet.

Als Trennmittel eignen sich z.B. optisch aktive Säuren, wie die D- und L-Formen von Weinsäuren, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Ferner ist die Trennung der Enantiomeren mit den an sich bekannten Methoden der präparativen Chromatographie möglich. Als stationäre Phase ist Kieselgel bevorzugt. Besonders bevorzugte Laufmittel sind Mischungen von Essigsäureethylester und Heptan bzw. Dichlormethan und Methanol.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich insbesondere zur Behandlung von Arrhythmien und von Tachykardien.

Dabei werden die erfindungsgemäßen Substanzen in der Regel in Analogie zu bekannten antiarrhythmisch wirksamen Substanzen wie Aprindin, Flecainid oder Amiodaron, vorzugsweise in Dosierungen zwischen etwa 1 und 100 mg, insbesondere zwischen 2 und 20 mg pro Dosierungseinheit verabreicht.

Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie an Kieselgel und/oder Kristallisation. Die optischen Drehwerte wurden in Methanol (c = 1) gemessen, sofern nichts anderes angegeben wird. Wird bei den einzelnen Beispielen die Entstehung von zwei Substanzen beschrieben, so liegen diese immer getrennt voneinander vor.

Vor- und nachstehend sind alle Temperaturen in Celsiusgraden angegeben.

**Beispiel 1**

Man löst 4 mmol 1-(2-p-Nitrophenyl-ethyl)-3-piperidinol [hergestellt aus 3-Piperidinol durch Alkylierung mit p-Nitrophenethylbromid oder p-Nitrostyrol], 4 mmol p-Nitrophenol, 4 mmol Triphenylphosphin und 4 mmol Diethylazodicarboxylat in 100 ml THF und rührt 48 Std. bei Raumtemperatur. Nach üblicher Aufarbeitung chromatographiert man den Rückstand über Kieselgel, wobei nacheinander Dichlormethan/Methanol (99:1) und Ethylacetat/Methanol (99:1) als Laufmittel verwendet werden.

Man erhält:

a) 1-(2-p-Nitrophenyl-ethyl)-2-(p-nitrophenoxy-methyl)-pyrrolidin;

b) 1-(2-p-Nitrophenyl-ethyl)-3-(p-nitrophenoxy)-piperidin.

**Beispiel 2**

Analog Beispiel 1 erhält man ausgehend von (-)-1-(2-p-Nitrophenyl-ethyl)-2-prolinol nach Chromatographie über Kieselgel unter Verwendung von Ethylacetat/Heptan (7:3) und anschließend Dichlormethan/Methanol (99:1) als Laufmittel:

a) (-)-1-(2-p-Nitrophenyl-ethyl)-2-(p-nitrophenoxy-methyl)-pyrrolidin
$[\alpha]_D^{20}$ -98,4°;

b) (+)-1-(2-p-Nitrophenyl-ethyl)-3-(p-nitrophenoxy)-piperidin
$[\alpha]_D^{20}$ +37,2°.

Analog erhält man
aus (+)-1-(2-p-Nitrophenyl-ethyl)-2-prolinol:

a) (+)-1-(2-p-Nitrophenyl-ethyl)-2-(p-nitrophenoxy-methyl)-pyrrolidin
$[\alpha]_D^{20}$ +97,8°;

b) (-)-1-(2-p-Nitrophenyl-ethyl)-3-(p-nitrophenoxy)-piperidin
$[\alpha]_D^{20}$ -36,6°.

**Beispiel 3**

Analog Beispiel 1 erhält man ausgehend von 1-(2-p-Nitrophenyl-ethyl)-2-hydroxymethylpiperidin durch Umsetzung mit p-Nitrophenol bei einer Reaktionszeit von 60 Std. (Reinigung: Kieselgel / Methyl-tert.-butylether und anschließend Heptan/Aceton (7:3))
1-(2-p-Nitrophenyl-ethyl)-2-(p-nitrophenoxy-methyl)-piperidin sowie
1-(2-p-Nitrophenyl-ethyl)-3-(p-nitrophenoxy)-hexahydroazepin.

Nachfolgende Umsetzung mit Fumarsäure liefert nach Kristallisation (Ethylacetat/Diisopropylether)

a) 1-(2-p-Nitrophenyl-ethyl)-2-(p-nitrophenoxy-methyl)-piperidin
F. (Fumarat) 122°;

b) 1-(2-p-Nitrophenyl-ethyl)-3-(p-nitrophenoxy)-hexahydro-azepin
F. (Fumarat) 137°.

**Beispiel 4**

Analog Beispiel 1 erhält man durch Umsetzung von p-Nitrophenol
mit 1-(2-p-Nitrophenyl-ethyl)-3-hydroxymethylpiperidin
(Reinigung: Kieselgel/Methyl-tert.-butylether und anschließend Heptan/Aceton (7:3)) das
1-(2-p-Nitrophenyl-ethyl)-3-(p-nitrophenoxy-methyl)-piperidin;
mit 1-(2-p-Nitrophenyl-ethyl)-4-piperidinol
(Reinigung: Kieselgel/Heptan:Aceton (7:3) und anschließend Ethylacetat/Methanol (19:1)) das
1-(2-p-Nitrophenyl-ethyl)-4-(p-nitrophenoxy)-piperidin;
mit (-)-1-(2-p-Nitrophenyl-ethyl)-3-pyrrolidinol
(Reinigung: Kieselgel/Methyl-tert.-butylether:Petrolether:Methanol (25:24:1)) das
(+)-1-(2-p-Nitrophenyl-ethyl)-3-(p-nitrophenoxy)-pyrrolidin, F. 97°,
$[\alpha]_D^{20}$ = +13,8° (Dioxan).

**Beispiel 5**

Analog Beispiel 1 erhält man durch Umsetzung von 1-Benzhydrylazetidin [erhältlich durch Umsetzung von Benzhydrylamin mit 1-Chlor-2,3-epoxypropan] mit N-(4-Hydroxyphenyl)-phthalimid nach üblicher Aufarbeitung das 1-Benzhydryl-3-[4-(1,3-dioxo-2-isoindolinyl)-phenoxy]-azetidin (Reinigung: Kieselgel/Diisopropylether:Methanol (49:1)).

Abspaltung der Benzhydrylgruppe durch Behandlung mit $H_2$-Gas/Pd-Kohle(Pd-Gehalt 1 %) in Toluol bei Raumtemperatur liefert 3-[4-(1,3-Dioxo-2-isoindolinyl)-phenoxy]-azetidin.

**Beispiel 6**

Man löst 1 mmol 3-[4-(1,3-Dioxo-2-isoindolinyl)-phenoxy]-azetidin in 40 ml Dichlormethan, fügt 1 Equivalent p-Nitrophenethylbromid hinzu und rührt 6 Std. bei Raumtemperatur. Nach Üblicher Aufarbeitung erhält man 1-(2-p-Nitrophenyl-ethyl)-3-[4-(1,3-dioxo-2-isoindolinyl)-phenoxy]-azetidin.

**Beispiel 7**

Man löst 0,9 g 1-(2-p-Nitrophenyl-ethyl)-3-[4-(1,3-dioxo-2-isoindolinyl)-phenoxy]-azetidin in 50 ml THF und fügt 0,5 g Hydrazin hinzu. Es wird 2 Std. gekocht und anschließend wie üblich aufgearbeitet. Man erhält 1-(2-p-Nitrophenyl-ethyl)-3-(p-aminophenoxy)-azetidin.

**Beispiel 8**

18 mmol p-Nitrophenol werden in 15 ml DMF gelöst, mit 22 mmol NaH versetzt und 30 min bei 40° gerührt. Anschließend werden 18 mmol 1-(2-p-Nitrophenyl-ethyl)-2-chlormethyl-piperidin [hergestellt aus dem Piperidinol-Derivat durch Halogenierung mit Thionylchlorid/DMF in Dichlormethan] in 20 ml DMF gelöst, zu der Phenolat-Lösung gegeben und 4 Std. bei 90° gerührt. Man engt die Lösung ein, nimmt den Rückstand in Toluol auf, wäscht mit 2 N Natronlauge und extrahiert mit 2 N Salzsäure. Nach üblicher Aufarbeitung und chromatographischer Reinigung (Kieselgel/Methyl-tert.-butylether) erhält man

a) 1-(2-p-Nitrophenyl-ethyl)-2-(p-nitrophenoxy-methyl)-piperidin;

b) 1-(2-p-Nitrophenyl-ethyl)-3-(p-nitrophenoxy)-hexahydro-azepin.

**Beispiel 9**

85 mmol 1-(2-p-Nitrophenyl-ethyl)-2-(p-nitrophenoxy-methyl)-pyrrolidin werden über 15 g Raney-Ni in 400 ml Ethanol und 40 ml THF, innerhalb 7 Std. bei 20° und 3 bar hydriert. Die Lösung wird eingeengt und wie üblich aufgearbeitet. Man erhält 1-(2-p-Aminophenyl-ethyl)-2-(p-aminophenoxymethyl)-pyrrolidin

Analog erhält man durch katalytische Reduktion mit Raney-Ni:

aus 1-(2-p-Nitrophenyl-ethyl)-3-(p-nitrophenoxy)-piperidin:
1-(2-p-Aminophenyl-ethyl)-3-(p-aminophenoxy)-piperidin;
aus (-)-1-(2-p-Nitrophenyl-ethyl)-2-(p-nitrophenoxy-methyl)-pyrrolidin:
(-)-1-(2-p-Aminophenyl-ethyl)-2-(p-aminophenoxy-methyl)-pyrrolidin;
aus (+)-1-(2-p-Nitrophenyl-ethyl)-3-(p-nitrophenoxy)-piperidin:
(+)-1-(2-p-Aminophenyl-ethyl)-3-(p-aminophenoxy)-piperidin;
aus (+)-1-(2-p-Nitrophenyl-ethyl)-2-(p-nitrophenoxy-methyl)-pyrrolidin:
(+)-1-(2-p-Aminophenyl-ethyl)-2-(p-aminophenoxy-methyl)-pyrrolidin;
aus (-)-1-(2-p-Nitrophenyl-ethyl)-3-(p-nitrophenoxy)-piperidin:
(-)-1-(2-p-Aminophenyl-ethyl)-3-(p-aminophenoxy)-piperidin;
aus 1-(2-p-Nitrophenyl-ethyl)-2-(p-nitrophenoxy-methyl)-piperidin:
1-(2-p-Aminophenyl-ethyl)-2-(p-aminophenoxy-methyl)-piperidin;
aus 1-(2-p-Nitrophenyl-ethyl)-3-(p-nitrophenoxy)-hexahydro-azepin:
1-(2-p-Aminophenyl-ethyl)-3-(p-aminophenoxy)-hexahydro-azepin;
aus 1-(2-p-Nitrophenyl-ethyl)-3-(p-nitrophenoxy-methyl)-piperidin:
1-(2-p-Aminophenyl-ethyl)-3-(p-aminophenoxy-methyl)-piperidin;
aus 1-(2-p-Nitrophenyl-ethyl)-4-(p-nitrophenoxy)-piperidin:
1-(2-p-Aminophenyl-ethyl)-4-(p-aminophenoxy)-piperidin;
aus (+)-1-(2-p-Nitrophenyl-ethyl)-3-(p-nitrophenoxyl)-pyrrolidin:
(+)-1-(2-p-Aminophenyl-ethyl)-3-(p-aminophenoxy)-pyrrolidin.

## Beispiel 10

13 mmol 1-(2-p-Aminophenyl-ethyl)-2-(p-aminophenoxy-methyl)-pyrrolidin werden in 40 ml getrocknetem Pyridin gelöst, unter Rühren, unter $N_2$-Atmosphäre und Eiskühlung werden bei einer Temperatur von 5-10° 39 mmol Mesylchlorid zugetropft und bei gleicher Temperatur 2 Std. gerührt. Die Lösung wird mit Ethylacetat versetzt. Der entstandene Niederschlag wird abgetrennt und in Ethylacetat / $NaHCO_3$-Lösung aufgenommen. Nach üblicher Aufarbeitung und chromatographischer Reinigung (Kieselgel / Dichlormethan / Methanol (49:1)) erhält man 1-(2-p-Methylsulfonamidophenylethyl)-2-(p-methylsulfonamidophenoxy-methyl)-pyrrolidin, F. 61-63°.

Analog erhält man durch Umsetzung mit Mesylchlorid:
aus 1-(2-p-Aminophenyl-ethyl)-3-(p-aminophenoxy)-piperidin:
1-(2-p-Methylsulfonamidophenyl-ethyl)-3-(p-methylsulfonamidophenoxy)-piperidin, F. 148-149°;
aus (-)-1-(2-p-Aminophenyl-ethyl)-2-(p-aminophenoxy-methyl)-pyrrolidin:
(-)-1-(2-p-Methylsulfonamidophenyl-ethyl)-2-(p-methylsulfonamidophenoxy-methyl)-pyrrolidin, $[\alpha]_D^{20}$ -73,9°;
aus (+)-1-(2-p-Aminophenyl-ethyl)-3-(p-aminophenoxy)-piperidin:
(+)-1-(2-p-Methylsulfonamidophenyl-ethyl)-3-(p-methylsulfonamidophenoxy)-piperidin, F. 168°, $[\alpha]_D^{20}$ +31,8°;
aus (+)-1-(2-p-Aminophenyl-ethyl)-2-(p-aminophenoxy-methyl)-pyrrolidin:
(+)-1-(2-p-Methylsulfonamidophenyl-ethyl)-2-(p-methylsulfonamidophenoxy-methyl)-pyrrolidin, $[\alpha]_D^{20}$ -72,0°;
aus (-)-1-(2-p-Aminophenyl-ethyl)-3-(p-aminophenoxy)-piperidin:
(-)-1-(2-p-Methylsulfonamidophenyl-ethyl)-3-(p-methylsulfonamidophenoxy)-piperidin, F. 168°, $[\alpha]_D^{20}$ -32,7°;
aus 1-((2-p-Aminophenyl-ethyl)-2-(p-aminophenoxy-methyl)-piperidin:
1-(2-p-Methylsulfonamidophenyl-ethyl)-2-(p-methylsulfonamidophenoxy-methyl)-piperidin;
aus 1-(2-p-Aminophenyl-ethyl)-3-(p-aminophenoxy)-hexahydro-azepin:
(1-(2-p-Methylsulfonamidophenyl-ethyl)-3-(p-methylsulfonamidophenoxy)-hexahydro-azepin;
aus 1-(2-p-Aminophenyl-ethyl)-3-(p-aminophenoxy-methyl)-piperidin:
1-(2-p-Methylsulfonamidophenyl-ethyl)-3-(p-methylsulfonamidophenoxy-methyl)-piperidin, F. 199-200°;
aus 1-(2-p-Aminophenyl-ethyl)-4-(p-aminophenoxy)-piperidin:
1-(2-p-Methylsulfonamidophenyl-ethyl)-4-(p-methylsulfonamidophenoxy)-piperidin, F. 182-183°;
aus (+)-1-(2-p-Aminophenyl-ethyl)-3-(p-aminophenoxy)-pyrrolidin:
(+)-1-(2-p-Methylsulfonamidophenyl-ethyl)-3-(p-methylsulfonamidophenoxy)-pyrrolidin, F. 199-200°, $[\alpha]_D^{20}$ +7,3° (Dioxan).

## Beispiel 11

10 mmol 1-(2-p-Aminophenyl-ethyl)-3-(p-aminophenoxy)-pyrrolidin werden in 45 ml getrocknetem Pyridin sowie 45 ml Essigsäureanhydrid gelöst und 24 Std. bei Raumtemperatur gerührt. Die Suspension wird unter Eiskühlung mit Wasser versetzt, 3 Std. bei Raumtemperatur gerührt, mit 2 N Natronlauge bis zur Trübung versetzt und wie üblich aufgearbeitet. Nach Umkristallisieren aus Methanol erhält man 1-(2-p-Acetamidophenyl-ethyl)-3-(p-acetamidophenoxy)-pyrrolidin, F. 213-214°.

Die nachfolgenden Beispiele betreffen pharmazeutische Zubereitungen.

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 mg eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \times 2\ H_2O$, 28,48 g $Na_2HPO_4 \times 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird in Ampullen abgefüllt, unter aseptischen Bedingungen lyophilisiert und steril verschlossen. Jede Ampulle enthält 10 mg Wirkstoff.

**Patentansprüche**

**1.** Cyclische Amin-Derivate der Formel I

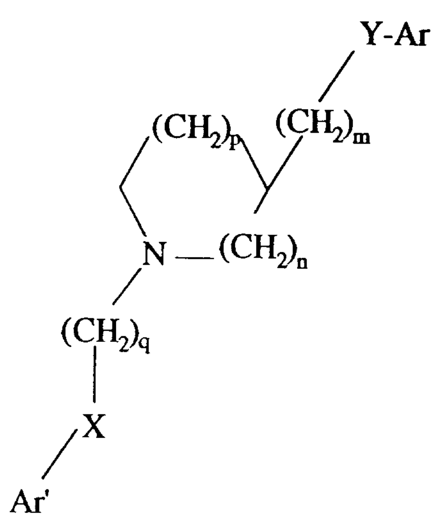

I,

worin

Ar und Ar' jeweils unabhängig voneinander unsubstituiertes oder ein- oder zweifach durch $NO_2$, $NH_2$, Hal, $CF_3$, A, $NHSO_2A$ oder NHAc substituiertes Phenyl,

| | |
|---|---|
| X und Y | jeweils unabhängig voneinander O oder eine Bindung, |
| m | 0 oder 1, |
| n | 0, 1 oder 2, |
| p | 0, 1, 2 oder 3, |
| q | 2 oder 3, |
| A | Alkyl mit 1-6 C-Atomen, |
| Hal | F, Cl, Br oder I und |
| Ac | Alkanoyl mit 1-8 C-Atomen, Aralkanoyl mit 8-10 C-Atomen oder Aroyl mit 7-11 C-Atomen, |

bedeuten,
sowie deren physiologisch unbedenkliche Salze.

**2.** Ein Enantiomer einer Verbindung der Formel I gemäß Anspruch 1.

**3.**

(a) 1-(2-p-Methylsulfonamidophenyl-ethyl)-3-p-methylsulfonamidophenoxy-piperidin;
(b) 1-(2-p-Methylsulfonamidophenyl-ethyl)-2-(p-methylsulfonamidophenoxy-methyl)-pyrrolidin;
(c) 1-(2-p-Nitrophenyl-ethyl)-3-p-nitrophenoxy-pyrrolidin;
(d) 1-(2-p-Acetamidophenyl-ethyl)-3-p-acetamido-phenoxy-pyrrolidin;
(e) 1-(2-p-Methylsulfonamidophenyl-ethyl)-3-p-methylsulfonamidophenoxy-pyrrolidin;
(f) 1-(2-p-Nitrophenyl-ethyl)-2-(p-nitrophenoxy-methyl)-piperidin;
(g) 1-(2-p-Nitrophenyl-ethyl)-3-p-nitrophenoxy-hexahydro-azepin;
(h) 1-(2-p-Methylsulfonamidophenyl-ethyl)-2-(p-methylsulfonamidophenoxy-methyl)-piperidin;
(i) 1-(2-p-Methylsulfonamidophenyl-ethyl)-3-p-methylsulfonamidophenoxy-hexahydro-azepin;
(j) 1-(2-p-Methylsulfonamidophenyl-ethyl)-4-p-methylsulfonamidophenoxy-piperidin;
(k) 1-(2-p-Methylsulfonamidophenyl-ethyl)-3-(p-methylsulfonamidophenoxy-methyl)-piperidin.

**4.** Verfahren zur Herstellung von Verbindungen der Formel I gemäß Anspruch 1, dadurch gekennzeichnet, daß man eine Verbindung der Formel II

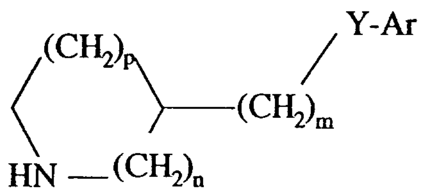

II,

worin
Ar, Y, m, n und p die angegebene Bedeutung haben,
mit einer Verbindung der Formel III,

$Ar\text{-}X\text{-}(CH_2)_q\text{-}L$ III,

worin
Ar, X und q die angegebene Bedeutung haben, und
L OH, Cl, Br oder eine reaktionsfähige, funktionell abgewandelte OH-Gruppe bedeutet,
umsetzt,
oder daß man zur Herstellung einer Verbindung der Formel I gemäß Anspruch 1, in der Y Sauerstoff bedeutet, eine Verbindung der Formel IV

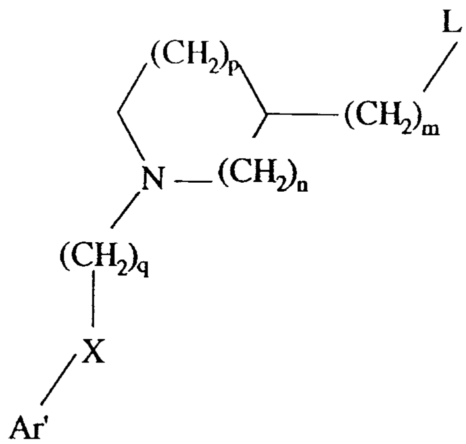

IV,

worin
Ar, X, L, m, n, p und q die angegebenen Bedeutungen besitzen,
mit einer Verbindung der Formel V

Ar-Z V,

worin
Ar die angegebene Bedeutung hat, und
- Z OH oder eine reaktionsfähige, funktionell abgewandelte OH-Gruppe, einschließlich einer Gruppe mit salzartigem Charakter bedeutet,

umsetzt,
oder daß man eine Verbindung der Formel Va

Ar'-Z Va,

worin Z und Ar' die angegebenen Bedeutungen haben,
mit einer Verbindung der Formel VI

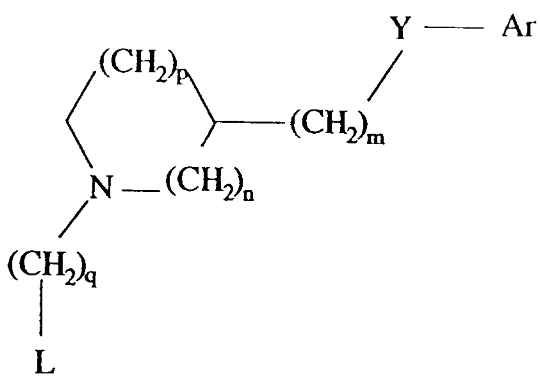

VI,

worin
Ar, Y, L, m, n, p und q die angegebenen Bedeutungen haben, umsetzt,
oder daß man eine Verbindung, die an sich der Formel I entspricht, aber anstelle einer oder mehrerer $CH_2$-Gruppen eine oder mehrere reduzierbare Gruppen besitzt, durch Reduktion in eine Verbindung der Formel I überführt und/oder daß man in einer Verbindung der Formel I eine oder beide Gruppen Ar bzw. Ar' in andere Reste Ar bzw. Ar' umwandelt und/oder daß man eine basische Verbindung der Formel I durch Behandeln mit einer Säure in eines ihrer physiologisch unbedenklichen Säureadditionssalze umwandelt.

**5.** Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

**6.** Pharmazeutische Zubereitung, gekennzeichnet durch einen Gehalt an mindestens einer Verbindung der Formel I und/oder einem ihrer physiologisch unbedenklichen Salze.

**7.** Verwendung von Verbindungen der Formel I nach Anspruch 1 oder von deren physiologisch unbedenklichen Salzen zur Herstellung eines Arzneimittels.

**8.** Verwendung von Verbindungen der Formel I nach Anspruch 1 oder von deren physiologisch unbedenklichen salzen bei der Bekämpfung von Krankheiten.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 94 11 5921

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | FR-A-2 681 319 (SYNTHELABO) 19. März 1993<br>* das ganze Dokument *<br>--- | 1,2,4-8 | C07D205/04<br>C07D207/08<br>C07D207/12<br>C07D211/22<br>C07D211/42<br>C07D211/46<br>C07D223/08<br>A61K31/445<br>A61K31/40<br>A61K31/55 |
| X | US-A-5 202 346 (BUTERA ET. AL.) 13. April 1993<br>* Ansprüche 1,2 *<br>* Spalte 1, Zeile 5 - Zeile 42 *<br>* Beispiel 4 *<br>--- | 1,2,4-8 | |
| X | JOURNAL OF MEDICINAL CHEMISTRY.,<br>Bd.35, 1992, WASHINGTON US<br>Seiten 4344 - 4361<br>* Tabellen I-III *<br>--- | 1,2,4-8 | |
| X | EP-A-0 494 717 (SHELL) 15. Juli 1992<br>siehe Ansprueche, Tabelle I<br>--- | 1,2,4 | |
| X | EP-A-0 307 121 (PFIZER) 15. März 1989<br>* das ganze Dokument *<br>--- | 1-8 | |
| X | EP-A-0 435 387 (SHELL) 3. Juli 1991<br>* Tabelle I *<br>* Ansprüche 1-4 *<br>---<br>-/-- | 1,2,4 | RECHERCHIERTE SACHGEBIETE (Int.Cl.6)<br>C07D |

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 30. Januar 1995 | Kissler, B |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 94 11 5921

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | EP-A-0 229 391 (EISAI) 22. Juli 1987<br>* Tabelle I *<br>* Anspruch 1 * | 1,2,4-8 | |
| X | EP-A-0 372 776 (PFIZER) 13. Juni 1990<br>* Beispiel 10 *<br>* Anspruch 1 * | 1,2,4-8 | |
| X | EP-A-0 094 595 (HOFMANN-LA ROCHE) 16. März 1983<br>* Anspruch 1 *<br>* Beispiel 24 * | 1,2,4-8 | |
| X | PATENT ABSTRACTS OF JAPAN<br>vol. 10, no. 303 (C-378) (2359) 16. Oktober 1986<br>& JP-P-61 115 068 (HOKURIKU SEIYAKU) 2. Juni 1986<br>* Zusammenfassung * | 1,2,4-8 | RECHERCHIERTE SACHGEBIETE (Int.Cl.6) |
| X | BULL. SOC. CHIM. FRANCE,<br>Nr.3, 1968<br>Seiten 987 - 999<br>Beispiele auf Seite 990 ff. | 1,2,4-8 | |
| X | J. PHARM. SCI.,<br>Bd.56, Nr.10, Oktober 1967<br>Seiten 1344 - 1347<br>* Tabelle III * | 1,2,4-8 | |

EPO FORM 1503 03.82 (P04C12)

EP 94 11 5921

-C-

UNVOLLSTÄNDIGE RECHERCHE

Vollständig recherchierte Patentanspruch: 3

Unvollständig recherchierte Patentansprüche: 1,2,4-8

Die Definition des/der folgenden Substituenten ist zu allgemein und/oder umfasst einen zu grossen Bereich von chemisch grundverschiedenen Resten und ist nur teilweise durch Beispiele in der Beschreibung gestützt:

Ar, Ar', m, n, p, q

Anspruch 1 konnte zwar vollständig on-line (STN, Registry und CA) recherchiert werden, doch die Recherche ergab zu viele relevante Dokumente und/oder Verbindungen, so dass der Recherchenbericht nicht als vollständig anzusehen ist.
Eine vollständige Liste aller relevanten Verbindungen in Form der Chemical Abstracts Registry Nummern und der CA Index Namen wurde von der Recherchenabteilung an die Prüfungsabteilung übermittelt.